# FASCICULE DE BREVET EUROPEEN

(11) **EP 1 095 642 B1**
(45) Date de publication et mention de la délivrance du brevet: **31.07.2002**
(21) Numéro de dépôt: 00402994.8
(22) Date de dépôt: 27.10.2000
(51) Int. Cl.: A61F 9/007

(54) **Appareil de phacoémulsification**
Vorrichtung zur Phako-Emulgierung
Phacoemulsification device

(30) Priorité: 28.10.1999 FR 9913528
(43) Date de publication de la demande: 02.05.2001
(73) Titulaire: Azar, Dimitri, Brooklin, MA 02445 (US); Hoang-Xuan, Thanh, 75015 Paris (FR)
(72) Inventeur: Azar, Dimitri, Brooklin, MA 02445 (US); Hoang-Xuan, Thanh, 75015 Paris (FR)
(74) Mandataire: Faber, Jean-Paul

(56) Documents cités:
- EP-A- 0 394 583
- DE-A- 3 822 011
- US-A- 4 168 707
- US-A- 4 184 510
- US-A- 5 268 624
- US-A- 5 328 456
- US-A- 5 810 765

## Description

La présente invention se rapporte à la phacoémulsification.

L'invention se rapporte à la chirurgie de la cataracte consistant à éliminer du sac capsulaire le cristallin et à remplacer ce dernier par une lentille.

L'invention vise plus particulièrement un appareil de phacoémulsification qui comprend une console avec des moyens de vérification du fonctionnement, un support pour un récipient d'un liquide destiné à l'irrigation et relié à une pièce à main, comportant un embout avec un orifice d'aspiration relié à une pompe volumétrique, ladite pièce à main étant directement reliée à un transducteur de manière que soient engendrées, à l'extrémité de la pièce à main, des vibrations ultrasoniques à fréquence variable de l'ordre de 27 et 64 KHz.

Cet ensemble est commandé par une pédale actionnée par le pied du chirurgien et qui comporte quatre positions, une position 0 dans laquelle toutes les fonctions sont arrêtées, une position 1 1 commandant uniquement l'irrigation, celle-ci étant commandée uniquement par la gravité. La position 2 commande l'irrigation et l'aspiration et la position 3, l'irrigation, l'aspiration et les ultrasons. Les ultrasons peuvent être réglés soit sur un mode linéaire, la puissance des ultrasons étant proportionnelle au déplacement de la pédale, soit en mode fixe.

Les principes de l'intervention sont les suivants : on pratique une incision dans la sclère ou la cornée afin d'accéder directement au cristallin à l'intérieur de son sac, on retire la capsule antérieure du sac, puis on engage l'embout de la pièce à main et en même temps qu'on procède à une irrigation permanente, on émulsifie et on aspire le cristallin selon différentes techniques dépendant du type de cataracte et des techniques du chirurgien. Une des méthodes, actuellement très utilisée, est la technique dite : "Divide and conquer", on creuse deux sillons en croix afin de former quatre quadrants qu'on isole les uns des autres.

Chaque quadrant est ensuite émulsifié par ultrasons et aspiré à travers la pièce à main. L'intervention présente une difficulté pour l'aspiration des morceaux des quadrants qui sont situés sous la pièce à main en regard de la partie à travers laquelle est insérée celle-ci. On est donc obligé d'utiliser un micro-manipulateur engagé dans le sac cristallinien et introduit dans la cornée en travers par rapport à la pièce à main. Le but de ce micro-manipulateur étant de présenter à l'embout de la pièce à main, les morceaux cataractés à émulsifier.

La technique antérieure dans le brevet US 4 184 510 prévoit deux pièces à main qui peuvent recevoir chacun deux des fonctions, celles-ci étant commandées par des pédales directement par le chirurgien, toutefois, il est prévu un sélecteur directement monté sur la machine et qui comporte trois positions, une position dans laquelle chaque pièce à main reçoit une irrigation aspiration et les ultrasons, une position dans laquelle l'une des pièces à main reçoit l'énergie ultrasonique et une position dans laquelle l'autre pièce à main reçoit ladite énergie ultrasonique.

Une telle disposition présente un certain nombre d'inconvénients. Le chirurgien, au cours de l'opération doit pouvoir commander sélectivement l'une ou l'autre des pièces à main en fonction de ce qui est de plus pratique afin d'éliminer le plus rapidement possible le cristallin. L'appareil, tel que prévu dans ce brevet, doit faire appel à une aide afin d'actionner le commutateur.

La technique antérieure du EP-A-394583 prévoit deux pièces à main, chacun ayant les trois fonctions irrigation, aspiration et ultrason, mais les moyens de commande ne sont pas décrits.

L'un des buts de la présente invention est de réaliser un appareil de phacoémulsification qui est destiné à améliorer l'efficacité de ladite phacoémulsification.

L'appareil, selon l'invention, est du type comprenant deux pièces à main reliées, chacune, à des moyens d'irrigation du sac capsulaire de l'oeil, chaque pièce à main comportant des moyens de vibrations ultrasoniques pour fragmenter le cristallin et étant reliée à des moyens d'aspiration, une console avec des moyens d'affichage et de vérification des différentes fonctions et des moyens de commande à trois positions correspondant à une première fonction d'irrigation du sac capsulaire par l'une ou par l'autre ou par les deux pièces à main, à une seconde fonction d'irrigation et d'aspiration par l'une ou par l'autre, ou par les deux pièces à main, à une troisième fonction pour l'irrigation, l'aspiration et l'émission de vibrations ultrasoniques par l'une ou par l'autre, ou par les deux pièces à main, ledit appareil étant caractérisé en ce qu'il comporte un support destiné à être posé sur le sol et sur lequel est mobile au moins une pédale actionnable par le pied et agencée de manière à commander les trois fonctions pour chaque pièce à main, soit indépendamment soit simultanément.

Grâce à une telle disposition, le chirurgien peut directement travailler soit simultanément des deux mains, soit séparément de la main gauche ou de la main droite aussi bien pour le creusage des quadrants, leur séparation et leur émulsification. Une telle disposition offre une grande sécurité.

Suivant une caractéristique de détail, le support comporte deux pédales dont l'une commande les fonctions de la première pièce à main, et la seconde les fonctions de la deuxième pièce à main, les pédales étant disposées pour être actionnées séparément ou simultanément.

Suivant une variante de réalisation, l'appareil peut comprendre, monté sur le support, un sélecteur susceptible d'occuper trois positions correspondant, la première à une position dans laquelle les différentes fonctions de la première pièce à main peuvent être actionnées, la seconde à une position dans laquelle seules les différentes fonctions de la seconde pièce à main peuvent être actionnées et la troisième à une position dans laquelle les différentes fonctions des deux pièces à main peuvent être actionnées simultanément.

Suivant encore une variante de réalisation, le support comporte un joint à la cardan relié à une extrémité de la pédale dont l'autre extrémité comporte deux tétons destinés à coopérer avec un bouton poussoir de commande des trois fonctions de chaque pièce à main, des moyens élastiques tendant à ramener la pédale dans une position neutre.

Enfin, suivant une dernière caractéristique, l'une au moins des pièces à main comporte un bouton poussoir de commande de l'arrêt des vibrations ultrasoniques. Cette disposition facilite encore le travail du chirurgien.

L'invention va maintenant être décrite avec plus de détails en se référant à des modes de réalisation particuliers donnés à titre d'exemple seulement et représentés aux dessins annexés, dans lesquels :

Figure 1 est une vue en perspective schématique d'un appareil, selon l'invention.

Figure 2 est une vue à plus grande échelle de la commande à pédales de l'appareil.

Figure 3 est une vue d'une variante de réalisation de la commande à pédale.

Figure 4 est une vue en élévation d'une pièce à main de l'appareil.

Figure 5 est une vue en perspective d'une variante de réalisation de la pédale.

Figure 6 est une vue en élévation de la pédale de la figure 5.

Figure 7 est une vue en élévation d'une variante de la pièce à main.

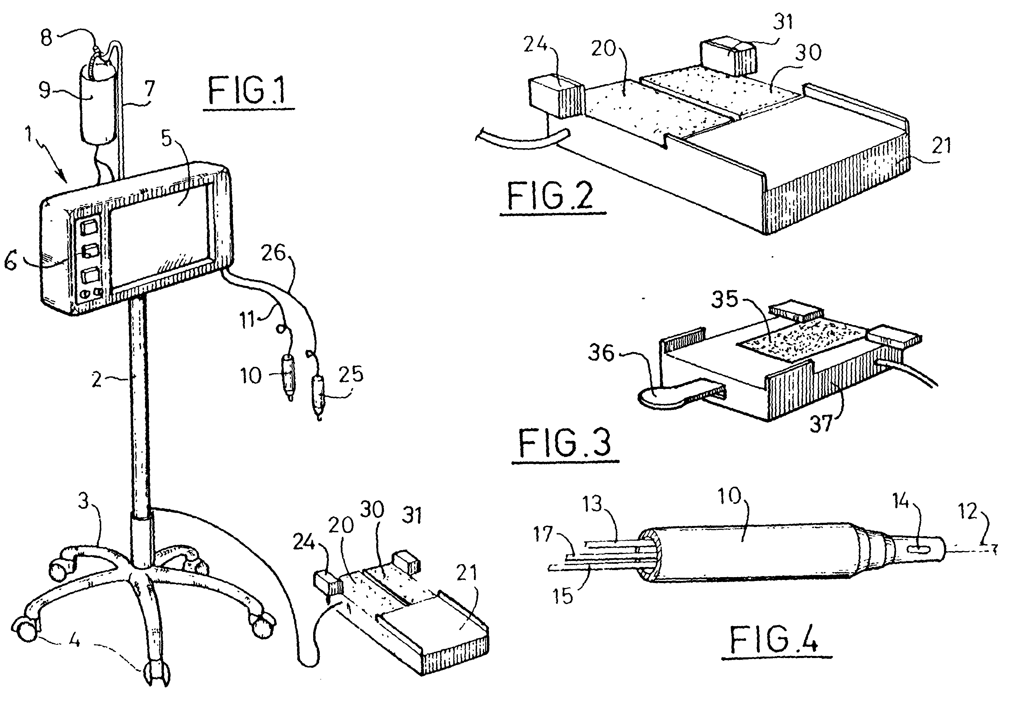

A la figure 1, on a représenté schématiquement une installation, selon l'invention.

Cette installation comprend une console 1 supportée par un montant 2 pourvu de pieds 3 avec des roulettes 4.

La console 1 comprend un écran permettant de visualiser les différentes fonctions et une série de boutons de commande 6.

Il est prévu un support 7 pourvu d'un crochet 8 supportant un flacon 9 contenant le liquide d'irrigation.

A partir de la console 1 s'étend une conduite 11 dont l'extrémité libre est reliée à une pièce à main 10.

La pièce à main 10 comporte un embout avec, à son extrémité libre, un orifice d'aspiration 12 relié à une conduite d'aspiration et au voisinage de cet orifice 12, deux ouvertures d'irrigation 14 reliées par une conduite 15 au flacon 9.

La pièce à main 10 est pourvue d'un transducteur relié par des conducteurs 17 à une source de courant électrique convenable de manière que ladite pièce à main puisse, à son extrémité, être animée de vibrations ultrasoniques.

La pièce à main 10 est commandée par une pédale 20 montée sur un support 21. Cette pédale peut être enfoncée plus ou moins et l'enfoncement correspond à trois positions qui sont repérables par le pied de l'utilisateur. La première position correspond à une irrigation, tandis que la position suivante correspond à l'irrigation et à l'aspiration et enfin la troisième position à l'irrigation, l'aspiration et les ultrasons.

Sur un côté du support 21, il peut être prévu un taquet 24 qui permet de bloquer la première fonction afin que l'irrigation soit continue.

L'appareil comprend une seconde pièce à main 25 exactement du même type que la pièce à main 10 et qui est reliée, à travers une conduite 26, à une source d'aspiration, au flacon 9 et à un générateur ultrasonique.

Cette pièce à main 25 est commandée par une pédale 30 portée par le support 21, cette pédale 30 a les mêmes fonctions que la pédale 20 et son enfoncement permet pour la pièce à main 25 les trois mêmes fonctions que celles de la pédale 20, soit irrigation, irrigation et aspiration et irrigation, aspiration et ultrasons.

Le support 21 est assez large de manière que le pied du chirurgien puisse commander séparément la pédale 20 ou la pédale 30 et éventuellement simultanément les deux pédales 20 et 30, son pied chevauchant lesdites deux pédales.

Le support 21 peut présenter un second taquet 31 permettant de bloquer la première fonction afin que l'irrigation soit continue.

Grâce à une telle disposition, le chirurgien peut, par deux incisions décalées angulairement, accéder au cristallin et travailler des deux mains tant pour le creusement des sillons que pour la nucléofracture, que pour l'émulsification progressive de tous les quadrants et sans avoir à utiliser un micro-manipulateur.

A la figure 3, on a représenté une variante de réalisation dans laquelle la commande ne comprend qu'une seule pédale 35, le support 37 de celle-ci comportant un sélecteur 36 manoeuvrable au pied et susceptible d'occuper trois positions, une position dans laquelle seule la pièce à main 10 est commandée, une position dans laquelle seule la pièce à main 25 est commandée et une position dans laquelle les pièces à main 10 et 25 sont commandées simultanément. Bien entendu, la pédale 35 actionne les trois fonctions comme les pédales 20 et 30.

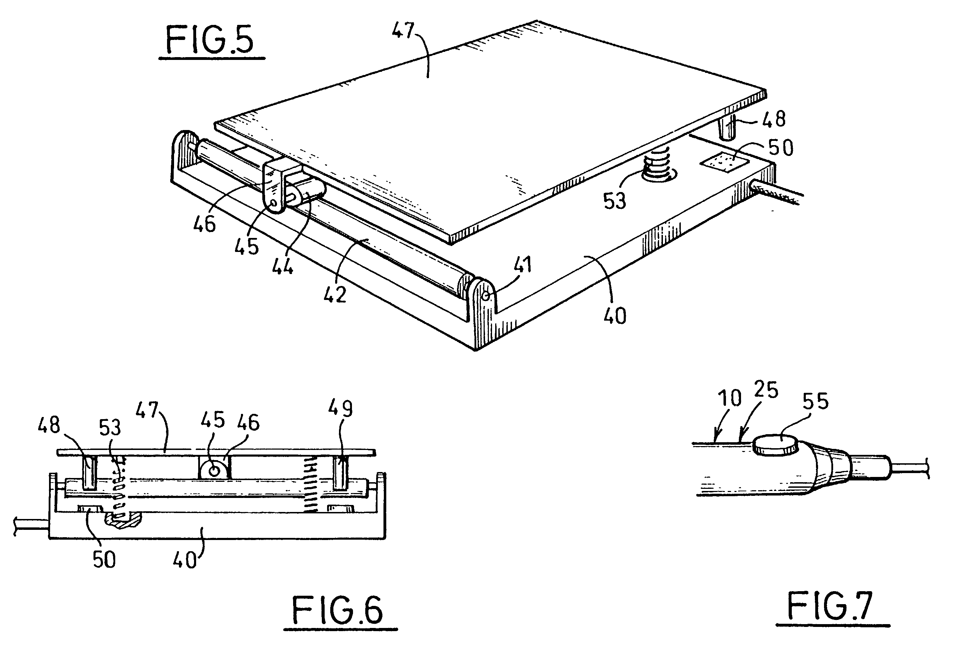

Aux figures 5 et 6, on a représenté une variante de réalisation de la pédale de commande.

Dans cette variante, il est prévu un support 40 à une extrémité duquel est monté un axe 41 sur lequel peut tourner un manchon 42 sur lequel est fixé, en un point intermédiaire médian de sa longueur et perpendiculairement, un élément tubulaire 44 dans lequel peut tourner un axe 45 relié, par une plaquette 46, à une extrémité d'une pédale 47. On réalise ainsi un joint à la cardan.

La pédale 47 comporte, à son extrémité libre, des tétons 48 et 49 destinés à coopérer respectivement avec des contacts à poussoir 50 et 51. Il est prévu deux ressorts de rappel 53 et 54 pour ramener la pédale 47 dans la position neutre.

On conçoit aisément que si on appuie du côté du bord de la pédale 47 adjacente au ressort 53, on commandera le contact 50, la pédale pivotant, d'une part sur l'axe 41 et, d'autre part, sur l'axe 45. Si on appuie sur le bord opposé, on commandera le contact 51. Si on appuie sur la partie centrale, on commandera les deux contacts.

Ainsi, on pourra commander, soit les trois fonctions de la pièce à main 10, soit les trois fonctions de la pièce à main 25, soit, simultanément, les trois fonctions des pièces à main 10 et 25.

On peut également prévoir (voir figure 7) que chaque pièce à main 10 et 25 comporte un bouton poussoir 55 permettant de bloquer l'émission des vibrations ultrasoniques.

Bien entendu, l'invention n'est pas limitée aux modes de réalisation qui viennent d'être décrits et représentés. On pourra y apporter de nombreuses modifications de détail sans sortir pour cela du cadre de l'invention, qui est defini par les revendications suivantes.

## Revendications

1. Appareil de phacoémulsification du type comprenant deux pièces à main (10, 25) reliées, chacune, à des moyens d'irrigation du sac capsulaire de l'oeil, chaque pièce à main (10, 25) comportant des moyens de vibrations ultrasoniques pour fragmenter le cristallin et étant reliée à des moyens d'aspiration, une console (5) avec des moyens d'affichage et de vérification des différentes fonctions et des moyens de commande à trois positions correspondant à une première fonction d'irrigation du sac capsulaire par l'une ou par l'autre ou par les deux pièces à main (10, 25), à une seconde fonction d'irrigation et d'aspiration par l'une ou par l'autre, ou par les deux pièces à main, à une troisième fonction pour l'irrigation, l'aspiration et l'émission de vibrations ultrasoniques par l'une ou par l'autre, ou par les deux pièces à main, ledit appareil comportant un support destiné à être posé sur le sol et sur lequel est mobile au moins une pédale actionnable par le pied et agencée de manière à commander les trois fonctions pour chaque pièce à main, soit indépendamment soit simultanément.

2. Appareil de phacoémulsification, selon la revendication 1, **caractérisé en ce que** le support (21) comporte deux pédales (20, 30) dont l'une commande les fonctions de l'une des pièces à main (10), et la seconde les fonctions de l'autre pièce à main (25), les pédales étant disposées de manière que le pied puisse les actionner séparément ou simultanément.

3. Appareil de phacoémulsification, selon la revendication 1, **caractérisé en ce que** le support (37) comporte un sélecteur (36) susceptible d'occuper trois positions correspondant la première, à une position dans laquelle les différentes fonctions de la première pièce à main (10) peuvent être actionnées, la seconde à une position dans laquelle, seules les différentes fonctions de la seconde pièce à main (25) peuvent être actionnées et la troisième position à une position dans laquelle les différentes fonctions des deux pièces à main peuvent être actionnées simultanément.

4. Appareil de phacoémulsification, selon la revendication 1, **caractérisé en ce que** le support (40) comporte un joint à la Cardan (42, 44) relié à une extrémité de la pédale (47) dont l'autre extrémité comporte deux tétons (48, 49) destinés à coopérer avec un bouton poussoir (50, 51) de commande des trois fonctions de chaque pièce à main, des moyens élastiques (53, 54) tendant à ramener la pédale dans une position neutre.

5. Appareil de phacoémulsification, selon la revendication 1, **caractérisé en ce que** l'une au moins des pièce à main comporte un bouton poussoir (55) permettant de bloquer l'émission de vibrations ultrasoniques.

## Patentansprüche

1. Vorrichtung zur Phako-Emulgierung, die zwei Handstücke (10, 25), die jedes mit Mitteln zum Spülen des Kapselsackes des Auges verbunden sind, wobei jedes Handstück (10, 25) Mittel für Ultraschallschwingungen aufweist, um die Kristallinse zu zerkleinern, und mit Saugmitteln verbunden ist, eine Konsole (5) mit Mitteln zur Anzeige und Kontrolle unterschiedlicher Funktionen sowie Mittel zur Steuerung dreier Schaltpositionen aufweist, die einer ersten Funktion des Spülens des Kapselsackes durch das eine oder durch das andere der zwei Handstücke (10, 25), einer zweiten Funktion des Spülens und des Saugens durch das eine oder das andere oder durch die beiden Handstücke, sowie einer dritten Funktion für das Spülen, das Saugen und das Abstrahlen von Ultraschallschwingungen durch das eine oder das andere oder durch die beiden Handstücke entsprechen, wobei genannte Vorrichtung einen Träger aufweist, der dazu bestimmt ist, auf dem Boden aufzuliegen und an dem zumindest ein Fußhebel beweglich ist, der durch den Fuß betätigbar und dazu eingerichtet ist, die drei Funktionen für jedes Handstück zu steuern, entweder unabhängig oder gleichzeitig.

2. Vorrichtung zur Phako-Emulgierung nach Anspruch 1, **dadurch gekennzeichnet, dass** der Träger (21) zwei Fußhebel (20, 30) aufweist, deren einer die Funktionen des einen Handstückes (10) und deren zweiter die Funktionen des anderen Handstückes (25) steuert, wobei die Fußhebel so angeordnet sind, dass der Fuß sie gesondert oder gleichzeitig betätigen kann.

3. Vorrichtung zur Phako-Emulgierung nach Anspruch 1, **dadurch gekennzeichnet, dass** der Träger (37) einen Wählschalter (36) aufweist, der drei Schaltstellungen einnehmen kann, von denen die erste einer Stellung entspricht, in der die verschiedenen Funktionen des ersten Handstückes (10) betätigt werden können, die zweite einer Stellung, in der lediglich die verschiedenen Funktionen des zweiten Handstückes (25) betätigt werden können, und die dritte Schaltstellung einer Stellung entspricht, in der die verschiedenen Funktionen der zwei Handstücke gleichzeitig betätigt werden können.

4. Vorrichtung zur Phako-Emulgierung nach Anspruch 1, **dadurch gekennzeichnet, dass** der Träger (40) ein Kardangelenk (42, 44) aufweist, das mit einem Ende des Fußhebel (47) verbunden ist, dessen anderes Ende zwei Zapfen (48, 49) aufweist, die zur Zusammenwirkung mit einem Drucktaster (50, 51) zur Steuerung der drei Funktionen jedes Handstükkes vorgesehen ist, wobei Federmittel (53, 54) den Fußhebel in eine Neutralstellung rückzustellen suchen.

5. Vorrichtung zur Phako-Emulgierung nach Anspruch 1, **dadurch gekennzeichnet, dass** zumindest eines der Handstücke einen Drucktaster (55) aufweist, der es ermöglicht, die Abstrahlung von Ultraschallschwingungen zu sperren.

## Claims

1. A phacoemulsification apparatus of the type comprising two hand pieces (10, 25) each connected to means for irrigating the capsular bag of the eye, each hand piece (10, 25) comprising ultrasonic vibration means for fragmenting the crystalline lens and being connected to aspiration means, a console (5) having means for displaying and checking the different functions and control means having three positions corresponding to a first function for irrigating the capsular bag by the one or the other hand piece or by both hand pieces (10, 25), corresponding to a second irrigation and aspiration function by the one or the other hand piece, or by both hand pieces, corresponding to a third function for the irrigation, aspiration and emission of ultrasonic vibrations by the one or by the other hand piece, or by both hand pieces,
the said apparatus comprising a support intended to be placed on the ground and on which there can move at least one pedal which can be operated by the foot and is designed so as to control the three functions for each hand piece, either independently or simultaneously.

2. A phacoemulsification apparatus according to Claim 1,
**characterised in that** the support (21) comprises two pedals (20, 30), the one of which controls the functions of one of the hand pieces (10), and the second of which controls the functions of the other hand piece (25), the pedals being disposed so that the foot may operate them separately or simultaneously.

3. A phacoemulsification apparatus according to Claim 1,
**characterised in that** the support (37) comprises a selector (36) capable of occupying three positions, the first corresponding to a position in which the different functions of the first hand piece (10) can be operated, the second corresponding to a position in which only the different functions of the second hand piece (25) can be operated and the third position corresponding to a position in which the different functions of the two hand pieces can be operated simultaneously,

4. A phacoemulsification apparatus according to Claim 1,
**characterised in that** the support (40) comprises a cardan joint (42, 44) connected to one end of the pedal (47), the other end of which comprises two studs (48, 49) intended to cooperate with a push button (50, 51) for controlling the three functions of each hand piece, elastic means (53, 54) tending to return the pedal into a neutral position.

5. A phacoemulsification apparatus according to Claim 1,
**characterised in that** at least the one of the hand pieces comprises a push button (55) enabling the emission of ultrasonic vibrations to be blocked.
